# EUROPÄISCHE PATENTSCHRIFT

(11) **EP 3 727 539 B1**
(45) Veröffentlichungstag und Bekanntmachung des Hinweises auf die Patenterteilung: **01.12.2021**
(21) Anmeldenummer: 18829314.6
(22) Anmeldetag: 18.12.2018
(51) Int. Cl.: A61M 16/04

(54) **TRACHEOSTOMIEVORRICHTUNG**
TRACHEOSTOMY DEVICE
DISPOSITIF DE TRACHÉOSTOMIE

(30) Priorität: 22.12.2017 DE 102017131172
(43) Veröffentlichungstag der Anmeldung: 28.10.2020
(73) Patentinhaber: Tracoe medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2018/085419
(87) Internationale Veröffentlichungsnummer: WO 2019/121633

(56) Entgegenhaltungen:
- WO-A1-99/33507
- US-A- 4 235 229
- US-A- 4 269 184
- US-A1- 2006 174 893

## Beschreibung

Die vorliegende Erfindung betrifft eine Tracheostomievorrichtung, insbesondere Tracheostomiekanüle oder Tracheostomieplatzhalter, mit einem Kanülenrohr, wobei das Kanülenrohr ein proximales Ende, ein distales Ende und eine Außenseite aufweist, wobei die Außenseite der Kanülenwand in einem näher an dem proximalen Ende liegenden Bereich oder in einem mittig zwischen dem proximalen Ende und distalen Ende angeordneten Bereich Rastelemente für die Fixierung eines Kanülenschildes aufweist.

Derartige Tracheostomievorrichtungen werden durch eine künstlich erzeugte Öffnung, ein sog. Tracheostoma, in die Trachea eines Patienten eingeführt. Das proximale Ende ist außerhalb des Patienten angeordnet und für den Patienten oder andere Personen, wie beispielsweise Pflegepersonen, zugänglich. Das distale Ende befindet sich in der Trachea. Das Kanülenrohr ist hohl oder massiv ausgeführt und dient der Verbindung zwischen dem proximalen Ende (außerhalb des Körpers angeordnet) und dem distalen Ende (innerhalb des Körpers angeordnet). Das die zwei Enden verbindende Kanülenrohr hält das Trachestoma offen und dient zusätzlich der Hindurchleitung von Atemgas (Atemluft) und/oder der Einführung von Absaugschläuchen, Sonden oder Instrumenten.

Durch die Tracheostomiekanüle kann ein Patient selbststätig ein- und ausatmen oder passiv beatmet werden. Während das meist gebogene Kanülenrohr der Tracheostomiekanüle mehrere Zentimeter in die Trachea eingeführt wird, füllt ein Tracheostomieplatzhalter oder Stomabutton lediglich das Stoma aus und ragt nur wenige Millimeter in die Trachea hinein. Tracheostomieplatzhalter und Stomabutton haben die Aufgabe, ein Zuwachsen des Tracheostoma zu verhindern, wenn eine Tracheostomiekanüle nicht durch das Tracheostoma geführt ist. Traecheostomieplatzhalter und Stomabutton können mit einem Stopfen verschlossen werden, sodass der Patient durch die natürlichen Atemwege atmen kann. Da bauartbedingt nur ein sehr geringer Anteil des Lumens der Trachea durch einen Tracheostomieplatzhalter bzw. Stomabutton versperrt ist, kann der Patient meist leichter ein- und ausatmen als bei einer in der Trachea befindlichen Tracheostomiekanüle. Tracheostomiekanülen haben dagegen Vorteile für beatmete Patienten. Im Unterschied zu Tracheostomiekanülen sind Tracheostomieplatzhalter bzw. Stomabutton aufgrund ihres Aufbaus nicht für künstliche Beatmungen vorgesehen. Sie werden nur für Patienten eingesetzt, die in der Lage sind spontan zu atmen. Häufig werden sie mittels Stopfen komplett verschlossen, damit der Patient über die natürlichen Atemwege atmet.

Bei herkömmlichen Tracheostomievorrichtungen kommt es außerdem nicht selten vor, dass das Kanülenrohr versehentlich verrutscht. Durch Hineinrutschen des distalen Endes weiter in die Trachea kann der Atemwiderstand enorm ansteigen, da die distale Öffnung gegen die Tracheawand drückt. Dies kann auch zu Druckstellen an der Trachea führen. Rutscht das distale Ende in den Stomakanal oder aus dem Stoma hinaus, kann es ebenfalls zu Atemproblemen kommen. In extremen Fällen besteht akute Erstickungsgefahr.

Um ein ungewolltes Lösen oder Verrutschen der Tracheostomievorrichtung in einem Tracheostoma zu verhindern, weisen bekannte Tracheostomievorrichtungen auf der Außenseite der Kanülenwand ein Kanülenschild auf, das mit dem Hals eines Patienten in Anlage gebracht werden kann. Das Kanülenschild kann mit den Rastelementen in Eingriff gebracht werden, wie es beispielsweise in der internationalen Patentanmeldung WO 99/33507 A1 gezeigt ist, und sichert so die Tracheostomievorrichtung gegenüber einem ungewollten Verrutschen. Die außenliegenden Rastelemente können jedoch häufig an den Rändern des Tracheostoma reiben, Verringern auf diese Weise den Tragekomfort und können Entzündungen verursachen. Ein weiteres Problem besteht darin, dass sich Bakterien und Schmutz im Bereich der Rastelemente sammeln und Entzündungen begünstigen können.

Häufig leiden Patienten auch unter dem Problem, dass der Durchmesser des Stomas deutlich größer als der Außendurchmesser der Tracheostomievorrichtung ist und ein Stopfen nicht die gewünschte Verschlusswirkung erzielt. Ein Großteil der Atemluft strömt dann weiter durch das Stoma und nicht durch die natürlichen Atemwege. Die durch das Stoma strömende Luft fehlt dem Patienten jedoch beim Sprechen. Beim Husten wird zudem zumindest ein Teil des Sekretes durch das Stoma und nicht durch die natürlichen Atemwege ausgeschieden. Dies führt häufig zu Entzündungen im Stoma. Aus diesen Gründen wird eine verbesserte Abdichtung zwischen Kanülenrohr und Stoma angestrebt.

Es ist daher eine Aufgabe der vorliegenden Erfindung eine Tracheostomievorrichtung mit einem erhöhten Tragekomfort und/oder einem geringeren Entzündungsrisiko bereitzustellen.

Zumindest eine dieser Aufgaben wird durch eine eingangs beschriebene Tracheostomievorrichtung gelöst, wobei ein die Außenseite des Kanülenrohrs umschließender Schlauch die Rastelemente abdeckt.

Der Schlauch bildet eine glatte außenliegende Mantelfläche, die ein Ansammeln von Bakterien oder Schmutz im Bereich der Rastelemente verhindert, sodass das Entzündungsrisiko bei der Verwendung der Tracheostomievorrichtung verringert ist. Die glatte Oberfläche des Schlauches verringert außerdem die Reibung zwischen den Rastelementen und den Rändern eines Tracheostomas, durch das die Tracheostomievorrichtung geführt ist.

Das Kanülenrohr der Tracheostomievorrichtung kann hohl oder massiv ausgeführt sein, wobei das proximale Ende und das distale Ende wahlweise offen oder geschlossen sein können. Ist das Kanülenrohr hohl und das proximale Ende sowie das distale Ende offen ausgeführt, kann die Tracheostomievorrichtung als Tracheostomiekanüle zur Hindurchführung von Atemluft verwendet werden. Eine Trachoestomievorrichtung mit geschlossenen Enden, oder auch mit offenen Enden, kann als Tracheostomaplatzhalter verwendet werden, um ein Zuwachsen des Tracheostomas zu verhindern, wenn eine Beatmung durch eine Tracheostomiekanüle vorübergehend nicht erforderlich ist. Die Tracheostomievorrichtung kann auch ein Sprechventil aufweisen. Das distale Ende des Kanülenrohrs kann einen Wulst oder einen Kragen aufweisen. Der Wulst oder Kragen hat einen im Vergleich zu dem Kanülenrohr größeren Außendurchmesser und ist auch größer als der Innendurchmesser des Tracheostoma durch das das distale Ende geführt werden soll. Ist das distale Ende durch ein Tracheostoma in die Trachea eingeführt, verhindert ein solcher Wulst oder Kragen, dass das Kanülenrohr ungewollt aus der Trachea herausrutscht. Der Wulst oder Kragen kann gegebenenfalls auch radial einziehbar sein, um das Einführen in das Tracheostoma und auch das Herausziehen zu erleichtern

Die Begriffe "distal" und "proximal" werden im Sinne der vorliegenden Erfindung aus der Sicht eines behandelnden Arztes verwendet, d.h. das proximale Ende der Tracheostomievorrichtung bzw. des Kanülenrohrs liegt außerhalb des Patientenkörpers, während das distale Ende im eingebrachten Zustand der Tracheostomievorrichtung im Inneren der Trachea angeordnet ist.

Die unter dem Schlauch angeordneten Rastelemente dienen der Befestigung eines Kanülenschildes, sodass die Tracheostomievorrichtung gegen ein ungewolltes Verrutschen in einem Tracheostoma gesichert werden kann.

In einer Ausführungsform bilden die Rastelemente ein sich in axialer Richtung des Kanülenrohrs wiederholendes Muster aus Vorsprüngen und Vertiefungen. Beispielsweise können die Rastelemente eine Mehrzahl von Vorsprüngen und Vertiefungen aufweisen, die in axialer Richtung des Kanülenrohrs in einer alternierenden Reihenfolge angeordnet sind.

Vorzugsweise weisen die Rastelemente mehrere, insbesondere zwischen zwei und zehn, in Umfangrichtung verlaufende und axial äquidistante Rippen (Vorsprünge) und dazwischen liegende Nuten (Vertiefungen) auf.

In einer Ausführungsform liegt der Grund der jeweiligen Nuten innerhalb einer gedachten Fortsetzung der Außenkontur des Kanülenrohrs und die Scheitel der Rippen sind außerhalb der gedachten Fortsetzung der Außenkontur angeordnet, wobei sich die gedachte Fortsetzung der Außenkontur von proximal an den äußeren Umfangs des an die Rastelemente anschließenden Abschnittes des Kanülenrohrs in direkter Linie bis zu dem äußeren Umfang des distal an die Rastelemente anschließenden Abschnittes des Kanülenrohrs erstreckt. Die gedachte Fortsetzung der Außenkontur des Kanülenrohrs bildet somit eine den Nutengrund der Rastelemente einhüllende Fläche. Vorzugsweise ist der Außendurchmesser der gedachten Fortsetzung der Außenkontur des Kanülenrohrs konstant. In einer Querschnittsansicht durch das Kanülenrohr ist der Nutengrund der jeweiligen Rastelemente innerhalb eines Durchmessers angeordnet, der dem Durchmesser der gedachten Fortsetzung der Außenkontur des Kanülenrohrs entspricht. Die Rippen ragen über den Außendurchmesser der gedachten Fortsetzung der Außenkontur des Kanülenrohrs hervor. Der radiale Abstand zwischen dem Nutengrund und der Scheitelfläche einer angrenzenden Rippe, die auch als die Höhe der Rippen bezeichnet werden kann, beträgt vorzugsweise 0,3 mm bis 2,5 mm.

In einer Ausführungsform entspricht die axiale Breite einer ringförmigen Nut dem ein- bis dreifachen, vorzugsweise dem ein- bis zweifachen, der axialen Breite einer umlaufenden Rippe. Vorzugsweise beträgt die axiale Breite 1 mm bis 5 mm (einschließlich).

Die Rastelemente können auch ein Gewinde oder eine Mehrzahl von rechteckigen, pyramidenförmigen, pyramidenstumpfförmigen oder teilkugelförmigen Vorsprüngen und/oder Vertiefungen aufweisen. Vorzugsweise sind die Abstände zwischen den Vorsprüngen und/oder Vertiefungen in axialer Richtung äquidistant. Mit dem sich in axialer Richtung des Kanülenrohrs wiederholenden Muster aus Rastelementen kann ein Kanülenschild in verschieden axialen Positionen an dem Kanülenrohr fixiert werden, sodass die axiale Position eines Kanülenschildes gegenüber dem Kanülenrohr im Rahmen des vorgegebenen Rasters einstellbar ist.

Der lichte Abstand zwischen den Vorsprüngen der Rastelemente entspricht der Breite der zwischen den Vorsprüngen angeordneten Vertiefungen und ist derart gewählt, dass der für das In Eingriff Bringen mit der Vertiefung vorgesehene Abschnitt des Kanülenschildes durch eine elastische Verformung des Schlauches in die Vertiefung eingreifen kann. Vorzugsweise entspricht der lichte Abstand zwischen den Vorsprüngen in axialer Richtung des Kanülenrohrs dem Platzbedarf des zum in Eingriff bringen in die Vertiefung vorgesehenen Abschnittes des Kanülenschildes zuzüglich der zweifachen Materialstärke des über die Vertiefung gespannten Schlauches.

In einer Ausführungsform erstrecken sich die Rastelemente mindestens über einen Umfangswinkelabschnitt von 20° um das Kanülenrohr herum. Die Rastelemente können sich auch über den gesamten Umfang des Kanülenrohrs erstrecken, sodass ein Kanülenschild an beliebigen Stellen entlang des Umfangs des Kanülenrohrs oder über dessen gesamten Umfang hinweg mit den Rastelementen in Eingriff gebracht werden kann. Die Rastelemente können sich in einer Variante über mindestens zwei einander diametral gegenüberliegenden Umfangswinkelabschnitte von jeweils 20° bis 60° erstrecken. Beispielsweise können zwei Abschnitte derart diametral gegenüberliegend angeordnet sein, dass bei einer in ein Tracheostoma eingesetzten Tracheostomievorrichtung Rastelemente lateral an dem Kanülenrohr angeordnet sind.

Der Begriff "lateral""wird aus der Sicht des zu behandelnden Patienten verstanden, bei dem eine Tracheostomievorrichtung durch ein Tracheostoma geführt ist. Folglich ist ein "lateraler" Abschnitt in einer Frontalansicht auf den Patienten der linken oder rechten Seite eines Patienten zugewandt.

Das Kanülenrohr, einschließlich der Rastelemente, hat in einer Ausführungsform im Querschnitt eine kreisförmige oder elliptische Grundform mit zwei diametral gegenüberliegend angeordneten abgeflachten Umfangsabschnitten, an bzw. in denen die Rastelemente angeordnet sind. Das Kanülenrohr kann in Richtung kaudal gebogen sein.

Der Begriff "kaudal" wird aus der Sicht des zu behandelnden Patienten verstanden, bei dem eine Tracheostomievorrichtung durch ein Tracheostoma geführt ist. Folglich ist ein "kaudaler" Abschnitt den unteren Körperteilen eines Patienten zugewandt. Entsprechend ist ein "kranialer" Abschnitt dem Kopf eines Patienten zugewandt.

Mit den Rastelementen kann ein Kanülenschild in Eingriff gebracht werden. In einer Ausführungsform umgreift eine Öffnung in einem mehr oder weniger scheibenförmigen Kanülenschild den Schlauch einschließlich der Rastelemente derart, dass der Rand der im Durchmesser vorzugsweise variablen Öffnung, zwischen axial benachbarten Rastelementen eingreift und dabei auch den Schlauch in den Raum zwischen Rastelementen eindrückt und so die axiale Position des Kanülenschildes gegenüber dem Kanülenrohr einstellbar fixiert. Das Kanülenschild kann mit der genügend groß eingestellten Öffnung über das Kanülenrohr geführt werden, sodass das Kanülenschild zumindest abschnittsweise das Kanülenrohr umgibt. Beispielsweise kann das Kanülenschild über das proximale Ende oder das distale Ende des Kanülenrohrs auf das Kanülenrohr bis in den Bereich des Kanülenrohrs mit den Rastelementen geschoben werden. In dem Bereich des Kanülenrohrs, in dem die Rastelemente angeordnet sind, kann die axiale Position des Kanülenschildes stufenlos oder in diskreten Schritten eingestellt und fixiert werden.

Beispielsweise weist das Kanülenschild in einer Ausführungsform ein Befestigungsmittel auf, das unter Verformung des Schlauches in die Rastelemente eingreift. Der Schlauch kann aus einem elastischen Kunststoff, vorzugsweise einem Silikon oder einem thermoplastischen Elastomer, hergestellt sein. Ein Schlauch aus einem elastischen Kunststoff ist leicht herstellbar, hygienisch und reduziert die Reibung zwischen der Tracheostomievorrichtung und einem Tracheostoma. Vorzugsweise ist der das Material des Schlauches ein thermoplastisches Copolyamid (ein TPA nach DIN EN ISO 18064:2014), ein thermoplastisches Polyesterelastomer (ein TPC nach DIN EN ISO 18064:2014), ein thermoplastisches Elastomer auf Olefinbasis (ein TPO nach DIN EN ISO 18064:2014), vorzugsweise ein Polypropylen oder ein Ethylen-Propylen-Dien), ein Styrol-Blockcopolymer (ein TPS nach DIN EN ISO 18064:2014), ein thermoplastisches Elastomer auf Urethanbasis (ein TPU nach DIN EN ISO 18064:2014) oder ein thermoplastisches Vulkanisat oder vernetztes thermoplastisches Elastomer auf Olefinbasis (ein TPV nach DIN EN ISO 18064:2014) oder ein Weich-PVC, oder ein Polypropylen oder ein Polyethylen.

In einer Ausführungsform weist der Schlauch eine mittlere Materialstärke von 0,01 mm bis 1 mm, vorzugsweise von 0,05 mm bis 0,4 mm, auf. Die Materialstärke ist derart ausgewählt, dass der Schlauch durch den Druck eines Befestigungsmittels am Kanülenschild beschädigungsfrei und reversibel in die zwischen den Vorsprüngen gebildeten Vertiefungen der Rastelemente einbringbar ist. Auf diese Weise kann, wie zuvor beschrieben, ein Befestigungsmittel des Kanülenschildes zusammen mit dem Schlauch in die Rastelemente eingreifen.

Das Befestigungsmittel kann, wie in einer weiteren Ausführungsform vorgesehen, dabei komplementär zu den Rastelementen ausgestaltet sein, wobei die Rastelemente vorzugsweise Hinterschneidungen und/oder Verriegelungsmittel zum in Eingriff bringen mit dem Befestigungsmittel aufweisen. Das Befestigungsmittel bildet einen Formschluss mit den Rastelementen, und fixiert so die axiale Position des Kanülenschildes gegenüber dem Kanülenrohr und ist auch wieder lösbar. Hinterschneidungen und Verriegelungsmittel verbessern die Befestigung des Kanülenschildes an dem Kanülenrohr und tragen dazu bei, dass die Befestigung nicht versehentlich durch einen unbeabsichtigten Stoß oder eine Bewegung des Patienten gelöst wird. Es versteht sich, dass das Befestigungsmittel zusätzlich oder alternativ zu den Rastelementen selbst Hinterschneidungen und/oder Verriegelungsmittel zum in Eingriff bringen mit den Rastelementen des Kanülenrohrs aufweisen kann.

In einer Ausführungsform wird das Befestigungsmittel durch Elemente am Rand der Öffnung des Kanülenschildes gebildet, durch die das Kanülenrohr zusammen mit dem Schlauch geführt ist. Beispielsweise weist der Rand der Öffnung zwei oder mehr gegenüberliegend zueinander angeordnete Vorsprünge auf, die unter Verformung des Schlauches in die unter dem Schlauch angeordneten Rastelemente eingreifen können. Vorzugsweise sind das Kanülenschild und die Öffnung derart elastisch verformbar, dass durch ein Zusammendrücken des Kanülenschildes von kaudal und kranial der seitliche lichte Abstand zwischen den Vorsprüngen vergrößert wird. Die Vorsprünge können außer Eingriff mit den Rastelementen des Kanülenrohrs gebracht und das Kanülenschild relativ zu dem Kanülenrohr verschoben werden. Sobald das Kanülenschild nicht mehr zusammengedrückt wird, verringert sich aufgrund der Elastizität des Schildes der lichte Abstand zwischen den Vorsprüngen und es kommt zu einem Formschluss zwischen den Vorsprüngen und den Rastelementen.

In einer weiteren Ausführungsform weist das Befestigungsmittel einen Druckknopf, einen Drehverschluss, einen um eine Achse schwenkbaren Hebel, ein Federelement und/oder einen Riegel auf. Solche Elemente tragen dazu bei, ein unabsichtliches Lösen des Kanülenschildes zu verhindern. In einer Ausführungsform weist der Schlauch einen proximalen Endabschnitt und einen distalen Endabschnitt auf, wobei der proximale Endabschnitt und der distale Endabschnitt proximal bzw. distal von den Rastelementen mit der Außenseite des Kanülenrohrs dicht verbunden, vorzugsweise verklebt oder verschweißt, sind. Der proximale Endabschnitt und der distale Endabschnitt bilden einen dichten Abschluss mit dem Kanülenrohr und verhindern, dass Bakterien und Schmutz zwischen den Schlauch und das Kanülenrohr, insbesondere zu den dort vorhandenen Rastelementen gelangen.

Die Endabschnitte bilden einen Übergang zu der Oberfläche des Kanülenrohrs, sodass das Kanülenrohr zusammen mit dem Schlauch leicht ein Stück weit in ein Tracheostoma eingeführt werden kann. Vorzugsweise erstrecken sich die Endabschnitte zwischen 2 mm bis 20 mm, bevorzugt mindestens 5 mm, über das jeweilige axiale Ende des Bereiches mit den Rastelementen hinaus in einen proximal bzw. distal an die Rastelemente anschließenden Bereich des Kanülenrohrs.

In einer Ausführungsform ist der distale Endabschnitt des Schlauches in einem Abstand von dem am weitesten distal angeordneten Rastelementen mit dem Kanülenrohr verbunden, der mindestens dem Außendurchmesser des Kanülenrohrs entspricht und bis zu 40 mm beträgt. Da das distale Ende bei einer ordnungsgemäßen Verwendung der Tracheostomievorrichtung im Stomakanal bzw. in der Trachea angeordnet ist, sind in diesem Bereich keine Rastelemente notwending.

Da das Kanülenrohr zwangsläufig einen kleineren Außendurchmesser aufweist als der Innendurchmesser eines Tracheostoma in das die Tracheostomievorrichtung eingesetzt werden soll, kann es geschehen, dass das Kanülenrohr auf seiner Außenseite das Tracheostoma nicht dicht verschließt. Therapieformen bei denen das Kanülenrohr beispielsweise mit Hilfe eines Stopfens bzw. eines Sprechventils komplett bzw. in Ausatemrichtung verschlossen werden, damit der Patient durch die natürlichen Atemwege atmet bzw. ausatmet, verlieren aufgrund der Undichtigkeit am Stoma an Effektivität oder werden unwirksam, wenn ein hoher Anteil der Atemluft durch das Stoma entweicht. Die Undichtigkeit wirkt sich folglich auch sehr negativ auf die Sprachqualität des Patienten aus. Große Undichtigkeiten machen ein Sprechen für einen Patienten unmöglich.

Ein weiteres Problem der Undichtigkeit besteht darin, dass der Patient nicht durch die natürlichen Atemwege abhustest sondern zumindest teilweise durch die Undichtigkeit im Stoma. Die häufige Kontamination des Stomas und der umgebenden Haut mit Sekret führt sehr häufig zu schwerwiegenden Entzündungen, die aufgrund der fortwährenden neuen Benetzung mit Bakterien enthaltendem Sekret nur sehr schwer abheilen.

Der distale Bereich des zentralen Schlauchabschnittes kann in einem solchen Fall zur Abdichtung genutzt werden, indem er dieser Bereich oder der gesamte zentrale Schlauchabschnitt gegenüber dem hiervon umfassten Kanülenrohr ein radiales Übermaß, gegebenenfalls auch ein axiales Übermaß aufweist. Der zentrale Bereich ist dann mit einem Fluid derart befüllbar, dass sich dieser distale Abschnitt gegenüber dem darunter befindlichen Kanülenrohr auf ein Übermaß ausdehnt und an die Wand des diesen umgebenden Tracheostomas anlegt. Der sich in das Tracheostoma hinein erstreckende Abschnitt des Schlauches schließt einen etwaigen Spalt zwischen Kanülenrohr und Wand des Tracheostomas und verhindert so, dass Luftoder Sekret vorbei durch das Tracheostoma hindurch gelangen.

Der zentrale Schlauchabschnitt kann mit einem Fluid befüllt sein und/oder mit einem Füllschlauch und/oder einem Ventil zum Befüllen des Schlauchabschnittes mit einem Fluid verbunden sein. Das Ventil kann zum Einstellen eines Fülldrucks bzw. zum Einstellen der Ausdehnung des zentralen Schlauchabschnittes verwendet werden. Das Volumen des distalen Bereichs zwischen Schlauch und Kanülenrohr kann auch durch eine innere Trennwand von dem übrigen Bereich des zentralen Abschnittes abgegrenzt sein. Das Ventil kann an dem freien Ende des Füllschlauches, d.h. an dem Ende des Füllschlauches, welches nicht mit dem zu füllenden Schlauch verbunden ist, angeordnet sein. Das Fluid kann beispielsweise ein Gel sein.

In einer Ausführungsform weist der befüllbare Schlauch bei einem Fülldruck in einem Bereich von höchstens 100 hPa, vorzugsweise in einem Bereich von 30 hPa bis 50 hPa, einen maximalen Außendurchmesser auf, der mindestens dem 1,5-fachen Außendurchmesser des darunter befindlichen Kanülenrohrs entspricht. Auf diese Weise kann durch eine Bestimmung des Innendruckes auf die Ausdehnung des Schlauches geschlossen werden und umgekehrt. Der auf den Stomakanal wirkende Druck kann somit durch eine Bestimmung des Innendruckes des Schlauches oder dessen Ausdehnung abgeschätzt werden, sodass Drucknekrosen im Stomakanal reduziert oder vermieden werden können.

In einer weiteren Ausführungsform ist der Schlauch ein High-Volume-Low-Pressure-Cuff. Diese Art von Cuff besteht aus einer flexiblen Weichfolie, die sich bei einer ordnungsgemäßen Verwendung nicht dehnt. Stattdessen ist die Größe des Cuffs so ausgewählt, dass er in einem freien, fluidgefüllten Zustand deutlich größer als der abzudichtende Hohlraum ist. Dies führt dazu, dass sich der im Hohlraum befindliche Cuff durch Befüllung mit einem Fluid unter Faltenbildung an die Wand des Hohlraums anschmiegt und abdichtet. Ein Vorteil von diesem Cuffdesign besteht darin, dass der messbare Cuffdruck dem Druck entspricht, mit dem di Außenhaut des Cuffs gegen die abzudichtende Wand drückt. Ein High-Volume-Low-Pressure-Cuff erreicht bereits bei einem vergleichsweise geringen Innendruck von 3 bis 10 hPa ein ausreichend großes Volumen, um den Stomakanal durch den das Kanülenrohr eingeführt ist ausreichend abzudichten. Für eine bessere Abdichtung kann der Innendruck des High-Volume-Low-Pressure-Cuffs aber auch deutlich erhöht sein. Beispielsweise kann der Innendruck 20 hPa betragen.

In einer Ausführungsform besteht der zentrale Schlauchabschnitt aus einer inneren Folie und einer elastischen äußeren Folie, wobei die innere Folie und die äußere Folie voneinander getrennt sind und die äußere Folie bei einem Innendruck von höchstens 100 hPa, vorzugsweise von höchstens 50 hPa, auf einen Durchmesser ausdehnbar ist, der mindestens das 1,5-fache des Außendurchmessers des unter dem zentralen Schlauchabschnitt angeordneten Kanülenrohrs beträgt, wobei die innere Folie aus einem elastischen Material mit einer geringeren elastischen Dehnung als die äußere Folie hergestellt ist und die innere Folie im Vergleich zu der äußeren Folie ein Übermaß aufweist. Beispielsweise kann die innere Folie einem High-Volume-Low-Pressure-Cuff entsprechen. Vorzugsweise weist der High-Volume-Low-Pressure-Cuff im Vergleich zu der äußeren Folie ein Übermaß auf und ist 30 % größer als der Durchmesser des zu verschließenden Stomas. Vorzugsweise ist die äußere Folie bei einem Innendruck von höchstens 100 hPa, vorzugsweise von höchstens 50 hPa, auf einen Durchmesser ausdehnbar, der maximal dem 2,5-fachen des Außendurchmessers des unter dem zentralen Schlauchabschnitt angeordneten Kanülenrohrs entspricht. Die Verwendung einer äußeren Folie und einer inneren Folie unterstützt ein weitestgehend faltenfreies Anlegen der äußeren Folie an die Wände des Stomakanals und verbessert die Abdichtung des Stomakanals. Die innere, im Vergleich zu der äußeren Folie festere Folie wirkt zudem als eine Stabilisierung, die ein gleichmäßiges Anlegen an und eine gleichmäßige Druckverteilung auf die Wand des Stomakanals erlaubt und zugleich verhindert, dass sich die Rastelemente durch die weiche, äußere Folie auf die Stomawand durchdrücken..

Im Allgemeinen wird die Position des Kanülenschildes beim ersten Anpassen einer Tracheostomievorrichtung ausgewählt und beibehalten, wobei aber die Position des Schildes auch mehrfach verstellt und/oder das Schild insgesamt von der Tracheostomievorrichtung abgenommen werden kann. Die meisten Ausführungsformen der vorliegenden Erfindung ermöglichen solch eine wiederholte Änderung der Position des Schildes entlang des Bereiches der Ratselemente, was aber in der Regel nicht notwendig ist. Manche der hier beschriebenen Ausführungsformen können auch für eine dauerhafte Fixierung einer einmal gewählten Position des Kanülenschildes verwendet werden.

In einer Ausführungsform unterteilt das fixierte Kanülenschild den zentralen Schlauchabschnitt in Umfangsrichtung reversibel, vorzugsweise weitestgehend fluid- oder gasdicht, in zwei Kammern. Eine erste Kammer ist proximal und eine zweite Kammer ist distal zu dem Kanülenschild angeordnet. Werden beide Kammern mit Fluid befüllt, so helfen sie das Kanülenschild in seiner axialen Position zu halten. Haben die mit Fluid befüllten Kammern einen größeren Querschnitt als die maximale Öffnung in dem Kanülenschild, durch die das Kanülenrohr geführt ist, kann das Kanülenschild nicht mehr über die befüllten Kammern hinweg verschoben werden. Falls jedoch das Fluid aus den Kammern entfernt werden kann, schrumpft der elastische Schlauch wieder auf seine ursprüngliche Größe und legt sich an das Kanülenrohr an, so dass dann das Schild wieder entlang der Kanüle bewegbar ist.

In einer Ausführungsform ist die Tracheostomievorrichtung ein Tracheostomieplatzhalter oder Stomabutton mit einem an dem distalen Ende angeordneten Kragen oder Wulst. Wie zuvor beschrieben, kann dieser einen im Vergleich zu dem Kanülenrohr größeren Außendurchmesser haben, der auch größer als der Innendurchmesser des Tracheostoma ist durch das das distale Ende geführt werden soll. Ist das distale Ende durch ein Tracheostoma in die Trachea eingeführt, verhindert ein solcher Kragen, dass das Kanülenrohr ungewollt aus der Trachea herausrutscht. Der Kragen kann gegebenenfalls auch radial einziehbar sein, um das Einführen in das Tracheostoma und auch das Herausziehen aus dem Tracheostoma zu erleichtern

In einer Ausführungsform ist der Kragen mit einem Fluid befüllbar und auf ein radiales Übermaß gegenüber dem Außendurchmesser des Kanülenrohrs an dem distalen Ende ausdehnbar. Nach dem Einführen des Tracheosstomieplatzhalter oder Stomabutton durch ein Stomakanal kann der befüllbare Kragen mit einem Fluid befüllt und auf ein Übermaß gegenüber dem Außendurchmesser des Kanülenrohrs an dem distalen Ende ausgedehnt werden, um so den Tracheostomieplatzhalter oder den Stomabutton gegenüber einem ungewollten Verrutschen zu sichern. Der auf ein Übermaß ausgedehnte Kragen hilft darüber hinaus, den Stomakanal gegenüber dem Kanülenrohr abzudichten.

Weist der Schlauch einen zentralen, befüllbaren Abschnitt auf, wie er zuvor beschrieben wurde, kann der Kragen zusammen mit dem zentralen Abschnitt des Schlauches oder getrennt von diesem befüllt werden. Vorzugsweise hat der befüllbare Kragen eine Materialstärke, die 3 bis 10-mal höher als die Materialstärke des zentralen Abschnittes des Schlauches ist.

Um ein ungewolltes Abknicken des Kragens in Richtung des distalen Ende des Kanülenrohrs zu erschweren, ist in einer Ausführungsform vorgesehen, dass distal des Kragens eine Materialverstärkung, vorzugsweise ein das Kanülenrohr umgebender Flansch, zum Abstützen des Kragens angeordnet ist. Der Flansch kann durch eine Vielzahl von in Umfangsrichtung des Kanülenrohrs verteilt angeordnete Vorsprünge oder durch einen durchgängigen Ring gebildet werden, wobei die Vorsprünge oder der Ring radial von dem Kanülenrohr hervorstehen. Die Materialverstärkung oder der Flansch sind im Vergleich zu dem Kragen formstabiler.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1: eine schematische Perspektivansicht einer Tracheostomievorrichtung gemäß einer ersten Ausführungsform der Erfindung,
- Figur 2: eine weggebrochene Teilansicht der Tracheostomievorrichtung gemäß Figur 1,
- Figur 3: eine schematische Detailansicht der Rastelemente von einer Tracheostomievorrichtung gemäß einer weiteren Ausführungsform der Erfindung,
- Figur 4: eine schematische Detailansicht der Rastelemente von einer Tracheostomievorrichtung gemäß einer weiteren Ausführungsform der Erfindung,
- Figur 5: eine Detailansicht eines Kanülenschildes von einer Tracheostomievorrichtung gemäß einer Ausführungsform der Erfindung,
- Figur 6: eine Detailansicht eines Kanülenschildes von einer Tracheostomievorrichtung gemäß einer Ausführungsform der Erfindung,
- Figur 7: eine Detailansicht einer Tracheostomievorrichtung gemäß einer weiteren Ausführungsform der vorliegenden Erfindung,
- Figur 8: eine Detailansicht einer Tracheostomievorrichtung gemäß einer noch weiteren Ausführungsform der vorliegenden Erfindung,
- Figur 9: eine schematische Perspektivansicht einer Tracheostomievorrichtung gemäß einer weiteren Ausführungsform der Erfindung,
- Figur 10: eine perspektivischer Längsschnitt durch eine Tracheostomievorrichtung gemäß einer weiteren Ausführungsform der Erfindung, und
- Figur 11: eine perspektivische Ansicht der Tracheostomievorrichtung gemäß Figur 10.

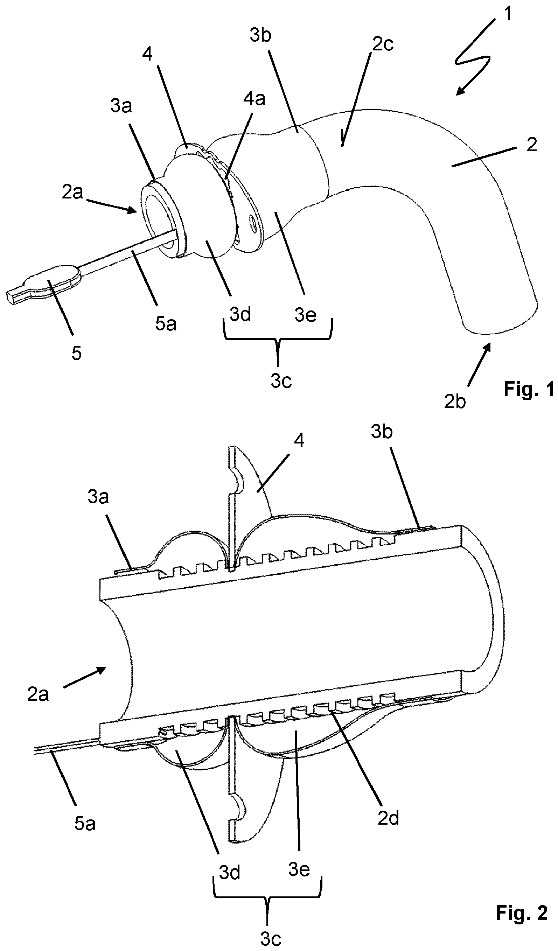

Die Figuren 1 und 2 zeigen eine Tracheostomievorrichtung gemäß einer ersten Ausführungsform der vorliegenden Erfindung in einer schematischen Perspektivansicht bzw. in einer weggebrochenen Teilansicht.

Die Tracheostomievorrichtung 1 hat ein Kanülenrohr 2 mit einem proximalen Ende 2a und einem distalen Ende 2b sowie mit einer Außenseite 2c. Die Tracheostomievorrichtung 1 der gezeigten Ausführungsform ist eine Tracheostomiekanüle mit einem gebogenen Kanülenrohr 2, wobei die im nachfolgend beschriebenen Merkmale hierauf nicht beschränkt sind. In einem Zustand, in dem das Kanülenrohr 2 durch ein Tracheostoma in die Trachea eines Patienten eingeführt ist, sind das distale Ende 2b in der Trachea und das proximale Ende 2a außerhalb der Trachea angeordnet. Durch das Kanülenrohr 2 kann der Patient mit Atemluft versorgt werden.

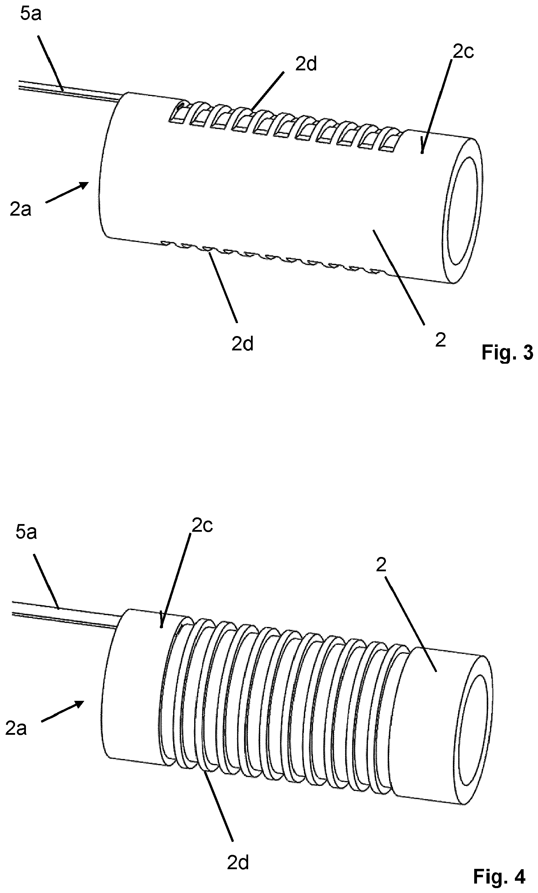

Im oberen Bereich, nahe der proximalen Öffnung 2a, befindet sich ein Schlauch 3, der, wie in der Figur 2 ersichtlich, auf der Außenseite der Kanülenwand 2c angeordnete Rastelemente 2d überdeckt. Die Rastelemente 2d bilden ein sich in axialer Richtung des Kanülenrohrs 2 wiederholendes Muster aus Vorsprüngen und Vertiefungen. Die Vorsprünge und Vertiefungen sind in Umfangsrichtung um das Kanülenrohr 2 verlaufende ringförmige Rippen und zwischen den Rippen angeordnete, ebenfalls ringförmige Nuten. Beispielhafte Modifikationen der Rastelemente 2d sind in den Figuren 3 und 4 dargestellt.

Der Abstand zwischen den Rippen ist derart gewählt, dass in die zwischen den Rippen gebildeten Nuten ein Kanülenschild 4 zusammen mit Wandabschnitten des Schlauches 3 eingreifen und so in dem betreffenden axialen Abschnitt des Kanülenrohrs 2 mit den Rastelementen 2d verrastet werden kann. Durch das sich in axialer Richtung wiederholende Muster der Rastelemente kann die axiale Position des Kanülenschildes 4 gegenüber dem Kanülenrohr 2 in diskreten Schritten eingestellt und fixiert werden. Die Schrittweite entspricht dem Wiederholabstand der ringförmigen Rippen.

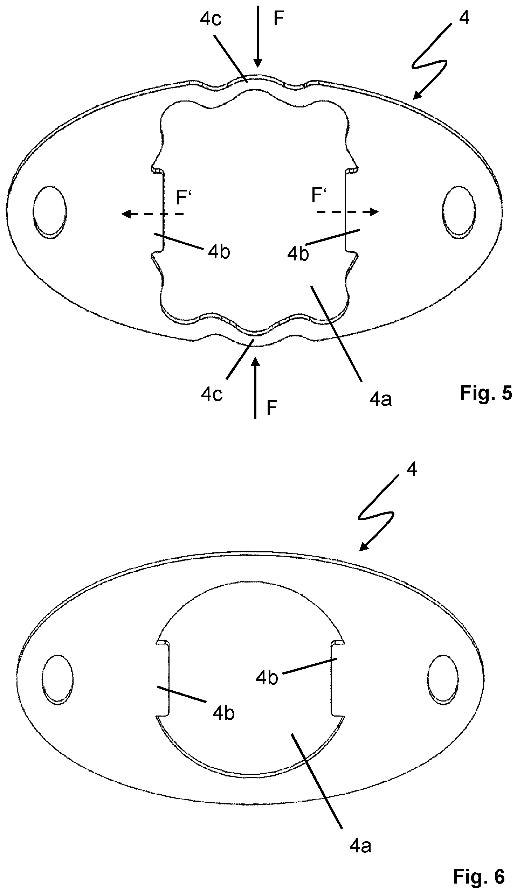

Das Kanülenschild 4 weist eine Öffnung 4a auf, durch die das Kanülenrohr 2, einschließlich des Schlauches 3 geführt werden kann. Die Öffnung 4a umgreift das Kanülenrohr 2, wobei sich die Ränder der die Öffnung 4a bildenden Ausnehmung des Kanülenschildes 4 in die ringförmigen Nuten der Rastelemente 2d erstrecken. Beispielhafte Ausführungsformen eines Kanülenschildes 4 sind in den Figuren 5 und 6 gezeigt.

Der die Rastelemente 2d überspannende Schlauch 3 ist außenliegend zu den Rastelementen 2d angeordnet und überdeckt auf diese Weise die Vorsprünge und Vertiefungen der Rastelemente 2d. Der Schlauch 3 verhindert, dass sich Bakterien, Fremdkörper oder Schmutz im Bereich der Rastelemente 2d anlagern können. Gleichzeitig bildet der Schlauch 3 eine glatte Oberfläche, die das Ein- und Ausführen der Tracheostomievorrichtung in bzw. aus einem Tracheostoma erleichtert ohne dass die Rastelemente 2d die Ränder des Tracheostoma reizen.

Der Schlauch 3 besteht aus einem elastischen Kunststoff, beispielsweise Silikon, und hat eine mittlere Materialstärke in einem Bereich von 0,1 mm bis 2 mm. Die Materialstärke des Schlauches 3, die Rastelemente 2d und das Kanülenschild 4 sind derart aufeinander abgestimmt, dass die Ränder 4b der die Öffnung 4a bildenden Ausnehmung des Kanülenschildes 4 unter einer elastischen Verformung des Schlauch 3 in die Rastelemente 2d eingreifen können.

Der Schlauch 3 hat einen proximalen Endabschnitt 3a und einen distalen Endabschnitt 3b sowie einen zentralen Abschnitt 3c, der den proximalen Endabschnitt 3a und den distalen Endabschnitt 3b miteinander verbindet. Der proximale Endabschnitt 3a erstreckt sich in einen Bereich des Kanülenrohrs 2 proximal von den Rastelementen 2d und der distale Endabschnitt 3b erstreckt sich in einen Bereich distal von den Rastelementen 2d. Die Bereiche, in denen der proximale Endabschnitt 3a und der distale Endabschnitt 3b mit dem Kanülenrohr 2 verbunden ist, sind in der Figur 2 gut zu erkennen.

Der proximale Endabschnitt 3a und der distale Endabschnitt 3b sind in Umfangsrichtung des Kanülenrohrs 2 mit der Kanülenwand 2c dicht verbunden. Der zentrale Abschnitt 3c des Schlauches 3 ist hingegen nicht an dem Kanülenrohr 2 oder den Rastelementen 2d befestigt. Der zentrale Schlauchabschnitt 3c ist mit einem Fluid derart befüllbar, dass er sich gegenüber dem darunter befindlichen Kanülenrohr 2 und den Rastelementen auf ein Übermaß ausdehnt, wie es in den Figuren 1 und 2 dargestellt ist. Zum Befüllen mit einem Fluid ist der Schlauch 3 über einen Füllschlauch 5a mit einer Befüllvorrichtung 5 verbunden.

Das mit den Rastelementen 2d in Eingriff stehende Kanülenschild 4 drückt die an das Kanülenschild angrenzenden Schlauchabschnitte in die Vertiefung zwischen den Rastelementen 2d und unterteilt so den zentralen Schlauchabschnitt in zwei Kammern 3d, 3e. Eine erste Kammer 3d liegt auf der proximalen Seite und eine zweite Kammer 3e liegt auf der distalen Seite des Kanülenschildes 4. Die distale Kammer 3e kann gegebenenfalls dazu beitragen, das Tracheostoma gegenüber dem Kanülenrohr 2 abzudichten.

In den Figuren 3 und 4 sind beispielhafte Varianten der Rastelemente 2d in einer Detailansicht ohne den die Rastelemente 2d überdeckenden Schlauch 3 gezeigt. Die Rastelemente 2d in der Figur 3 erstrecken sich über zwei diametral gegenüberliegend zueinander auf der Außenseite der Kanülenwand 2c angeordnete Umfangswinkelabschnitte von jeweils etwa 50°. Die Rastelemente der Umfangswinkelabschnitte bilden jeweils ein sich in axialer Richtung des Kanülenrohrs 2 wiederholendes Muster aus in Umfangsrichtung verlaufenden Rippen und Nuten auf.

Die in der Figur 4 gezeigten Rastelemente 2d erstrecken sich vollständig um den Umfang des Kanülenrohrs 2, wobei ringförmige Rippen und Nuten alternierend in axialer Richtung angeordnet sind. Der Wiederholabstand zwischen den Rippen und Nuten ist konstant. Die axiale Breite der ringförmigen Nuten entspricht dem zwei- bis vierfachen der axialen Breite einer Rippe. Die Nutbreite richtet sich nach der axialen Dicke des Schildes und der Wandstärke und Flexibilität des Schlauches und ist so bemessen, dass die Nut das Befestigungsmittel am Schild 4b und die an diesem beidseitig anliegenden Schlauchabschnitte aufnehmen kann, wie es beispielhaft in den Figuren 5 und 6 gezeigt ist.

Die Figuren 5 und 6 zeigen jeweils eine beispielhafte Ausführungsform eines Kanülenschildes 4, wie es mit den Rastelementen 2d einer erfindungsgemäßen Tracheostomievorrichtung 1 in Eingriff gebracht werden kann. Die Kanülenschilder 4 weisen eine Öffnung 4a auf, durch die ein Kanülenrohr 2 einer Tracheostomievorrichtung 1 hindurchgeführt werden kann. Die Öffnung 4a hat einen umlaufenden Rand, der die Öffnung 4a begrenzt. Auf zwei gegenüberliegenden Seiten weist der Rand jeweils einen Vorsprung auf, der als Befestigungsmittel 4b zum Befestigen des Kanülenschildes 4 mit den Rastelementen 2d dient.

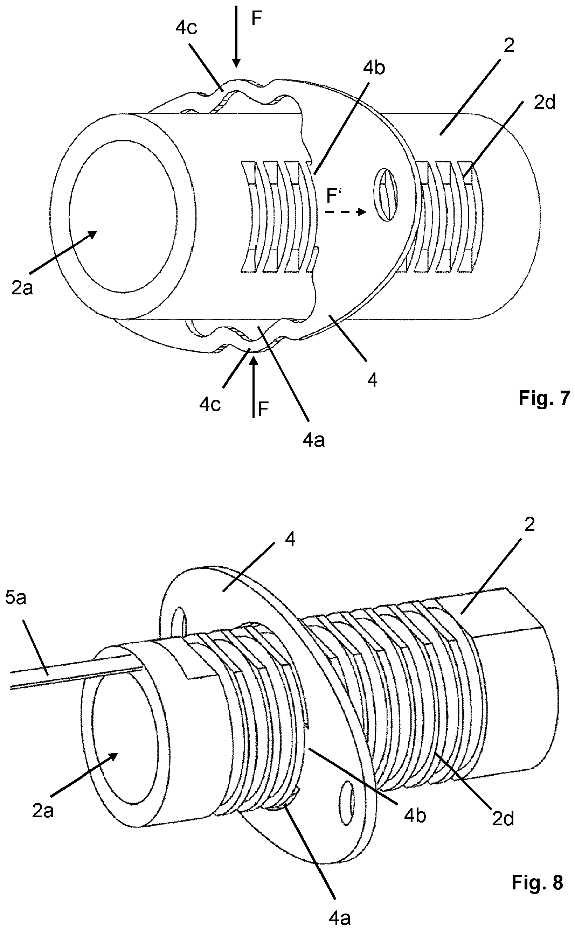

Der Querschnitt der Öffnung 4a des in der Figur 5 dargestellten Kanülenschildes 6 ist einer rechteckigen Form angenähert. Auf zwei gegenüberliegenden Seiten der Öffnung 4a sind zwei Vorsprünge als Befestigungsmittel 4b zum in Eingriff bringen mit den Rastelementen 2d vorgesehen. Auf den beiden anderen Seiten weist die Öffnung 4a zwei nach Außen vorspringende Ausbuchtungen 4c auf. Das Kanülenschild 4 kann durch ein beidseitiges Angreifen von außen im Bereich der Ausbuchtungen 4c mit zwei oder mehr Fingern gehalten werden. Durch Zusammendrücken im Bereich der Ausbuchtungen 4c in der angedeuteten Richtung F weitet sich der Querschnitt der Öffnung 4a in Richtung F' der beiden Vorsprünge 4b. Der lichte Abstand zwischen den in die Öffnung 4a hineinragenden Vorsprüngen 4b vergrößert sich und das Kanülenschild 4 kann verschoben werden, wenn es wie in der Figur 7 auf einem Kanülenrohr 2 angeordnet ist. Sobald das elastische Kanülenschild 4c entlastet wird, kehrt das Kanülenschild 4 wegen seiner Elastizität in seine Ausgangsform zurück. Das heißt, der lichte Abstand zwischen den Vorsprüngen 4b verringert sich wieder, sodass die Vorsprünge 4b wieder in Eingriff mit den Rastelementen 2d stehen. Das Kanülenschild 4 ist in seiner Position relativ zu dem Kanülenrohr 2 fixiert.

Der Querschnitt der Öffnung 4a des in der Figur 6 dargestellten Kanülenschildes 4 ist in etwa kreisrund mit zwei in Richtung des Mittelpunktes des Kreises vorspringenden Vorsprüngen 4b. Durch Drehen des Kanülenschildes können die Rastelemente außer Eingriff gebracht werden, sodass das Kanülenschild verschoben werden kann. Zur Arretierung wird das Kanülenschild wieder zurückgedreht, sodass die Vorsprünge 4b wieder mit den Rastelementen in Eingriff gebracht werden.

Die Figuren 7 und 8 zeigen jeweils in einer Detailansicht ein Kanülenschild 4, welches mit Rastelementen 2d eines Kanülenrohrs 2 in Eingriff gebracht ist, wobei der besseren Anschaulichkeit wegen der die Rastelemente 2d abdeckende Schlauch 3 nicht dargestellt ist.

Das in der Figur 7 gezeigte Kanülenschild 4 entspricht dem im Zusammenhang mit der Figur 5 dargestellten. Das Kanülenschild 4 hat eine Öffnung 4a, die einer Rechteckform angenähert ist, und zwei gegenüberliegenden angeordnete Befestigungsmittel 4b in Form von Vorsprüngen, die sich in die Öffnung 4a hinein erstrecken. Durch Zusammendrücken im Bereich der Ausbuchtungen 4c in die mit dem Bezugszeichen F angedeutete Richtung weitet sich der Abstand zwischen den Befestigungsmitteln 4, sodass die Vorsprünge außer Eingriff mit den durch die Rastelemente gebildeten Nuten gebracht werden.

Die Rastelemente 2d erstrecken sich über zwei diametral gegenüberliegend angeordnete Umfangswinkelabschnitte und bilden ein sich in axialer Richtung des Kanülenrohrs 2 wiederholendes Muster aus sich in Umfangsrichtung teilweise erstreckenden Rippen und Nuten. Die axiale Breite der Nut entspricht dem zwei- bis dreifachen der axialen Breite einer Rippe und reicht aus, das Schild und angrenzende Schlauchabschnitte darin aufzunehmen.

Das in der Figur 8 gezeigte Kanülenschild 4 entspricht dem im Zusammenhang mit der Figur 6 beschriebenen Kanülenschild 4. Die als Vorsprünge ausgestalteten Befestigungsmittel 4b ragen in die Öffnung 4a des Kanülenschildes 4 hinein und greifen in die Vertiefung der Rastelemente 2d eines durch die Öffnung 4a geführten Kanülenrohrs 2 ein. Die Rastelemente 2d bilden ein sich in axialer Richtung des Kanülenrohrs 2 wiederholendes Muster aus alternierend angeordneten Rippen und Nuten. Die Rippen sind in zwei gegenüberliegend zueinander angeordneten Umfangswinkelabschnitten abgeflacht, sodass in diesem Bereich der Querschnitt des Kanülenrohrs 2 einschließlich der Rastelemente 2d reduziert ist und von einer Kreisform abweicht. Durch Verdrehen des Kanülenschildes 4 können die Befestigungsmittel 4b in den abgeflachten Sektor des Kanülenrohres 2 bewegt werden, sodass das Kanülenschild 4 insgesamt in axialer Richtung des Kanülenrohrs 2 bewegt werden kann, ohne dass die Befestigungsmittel in einem Eingriff mit Rastelementen 2d stehen.

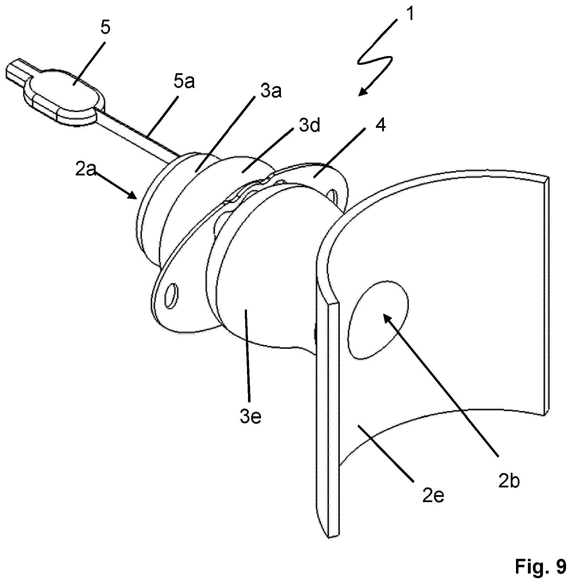

In der Figur 9 ist eine weitere Ausführungsform einer Tracheostomievorrichtung 1 gemäß der vorliegenden Erfindung dargestellt. Die Tracheostomievorrichtung 1 ist hier als ein sogenannter Tracheostomiieplatzhalter ausgeführt und unterscheidet sich von der in den Figuren 1 und 2 gezeigten Tracheostomievorrichtung 1 lediglich in der Form und Ausgestaltung des Kanülenrohrs 2, das in diesem Fall nicht zur Beatmung vorgesehen ist. Im Unterschied zu Tracheostomiekanülen, deren distales Ende mehrere Zentimeter in das Innere der Trachea eindringt, endet das distale Ende von Tracheostomieplatzhaltern bzw. Stomabuttons typischerweise am Eingang zur Luftröhre.

Das Kanülenrohr 2 der in der Figur 7 dargestellten Ausführungsform ist im Vergleich zu der in den Figuren 1 und 2 gezeigten Ausführungsform kürzer und gerade ausgestaltet. An seinem distalen Ende 2b weist das Kanülenrohr 2 einen Kragen 2e auf. Der Kragen 2e wird zusammen mit dem distalen Ende 2b durch ein Tracheostoma in die Trachea eines Patienten eingeführt. Für diesen Zweck kann der Kragen 2e elastisch verformt werden. In der Trachea nimmt der elastische Kragen 2e seine Ausgangsform an und hilft so die Position des distalen Endes der Tracheostomievorrichtung 1 in der Trachea zu fixieren. Zusätzlich hilft der Kragen das Stoma gegenüber dem Kanülenrohr abzudichten. Der Tracheostomieplatzhalter bzw. Stomabutton dient häufig zum vorübergehenden Verschließen eines nicht mehr oder vorübergehend nicht mehr benötigten Tracheostomas, wenn der Patent über die natürlichen Atemwege atmen kann.

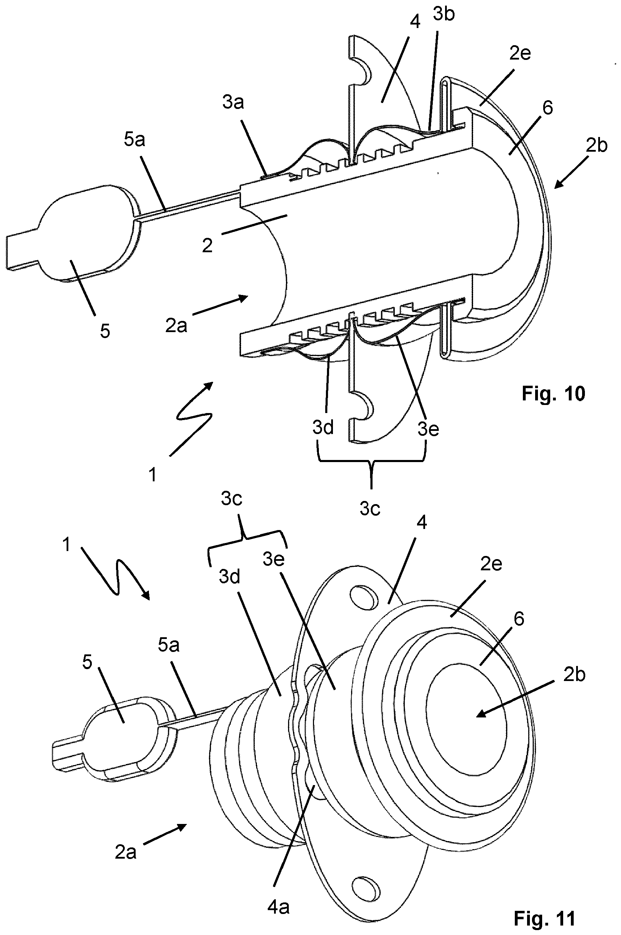

In den Figuren 10 und 11 ist eine weitere Ausführungsform einer Tracheostomievorrichtung gezeigt. Die Tracheostomievorrichtung ist ein Tracheostomieplatzhalter bzw. Stomabutton mit einem gerade verlaufenden Kanülenrohr 2. Das Kanülenrohr 2 hat ein proximales Ende 2a, ein distales Ende 2b und eine Außenseite 2c. Wie zuvor im Zusammenhang mit dem übrigen Ausführungsformen beschrieben, weist die Außenseite 2c Rastelemente für die Fixierung eines Kanülenschildes 4 auf, wobei ein die Außenseite 2c des Kanülenrohrs umschließender verformbarer Schlauch 3 die Rastelemente 2d abdeckt. Das Kanülenschild 4, die Rastelemente 2d sowie der die Rastelemente 2d überdeckende Schlauch 3 entsprechen der in der Figur 2 gezeigten Ausführungsform.

Am distalen Ende 2b ist ein Kragen 2e angeordnet, der mit einem Fluid befüllbar und auf ein radiales Übermaß gegenüber dem Außendurchmesser des Kanülenrohrs 2 an dem distalen Ende 2b ausdehnbar ist. In den Figuren 10 und 11 ist der Kragen 2e in einem befüllten und von dem Kanülenrohr 2 abstehenden Zustand gezeigt. Der Kragen 2e ist zusammen mit dem zentralen Abschnitt 3c des Schlauches 3 mit einem Fluid befüllbar und weist eine Materialstärke auf, die 3 bis 10-mal höher ist als die Materialstärke des zentralen Abschnittes 3c des Schlauches 3. Um ein versehentliches Abknicken des befüllten Kragens 2e in Richtung des distalen Endes 2b des Kanülenrohrs 2 zu verhindern bzw. zu erschweren, ist distal des Kragens 2d ein das Kanülenrohr 2 umgebender Flansch 6 zum Abstützen des Kragens 2e angeordnet.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Tracheostomievorrichtung
- 2: Kanülenrohr
- 2a: proximales Ende des Kanülenrohrs
- 2b: distales Ende des Kanülenrohrs
- 2c: Außenseite des Kanülenrohrs
- 2d: Rastelemente
- 2e: Kragen/Wulst
- 3: Schlauch
- 3a: proximaler Endabschnitt des Schlauches
- 3b: distaler Endabschnitt des Schlauches
- 3c: zentraler Abschnitt des Schlauches
- 3d: erste Kammer des Schlauches
- 3e: zweite Kammer des Schlauches
- 4: Kanülenschild
- 4a: Öffnung in dem Kanülenschild
- 4b: Befestigungsmittel des Kanülenschildes
- 4c: Ausbuchtungen
- 5: Befüllvorrichtung
- 5a: Füllschlauch
- 6: Flansch

## Patentansprüche

1. Tracheostomievorrichtung (1), insbesondere Tracheostomiekanüle oder Tracheostomieplatzhalter, mit einem Kanülenrohr (2), wobei das Kanülenrohr (2) ein proximales Ende (2a), ein distales Ende (2b) und eine Außenseite (2c) aufweist, wobei die Außenseite (2c) des Kanülenrohres (2) in einem mittig zwischen dem proximalen Ende (2a) und dem distalen Ende (2b) oder näher an dem proximalen Ende angeordneten Bereich Rastelemente (2d) für die Fixierung eines Kanülenschildes (4) aufweist, **dadurch gekennzeichnet, dass** ein die Außenseite (2c) des Kanülenrohres (2) umschließender verformbarer Schlauch (3) die Rastelemente (2d) abdeckt.

2. Tracheostomievorrichtung (1) nach Anspruch 1, **dadurch gekennzeichnet, dass** der Schlauch (3) einen proximalen Endabschnitt (3a) und einen distalen Endabschnitt (3b) aufweist, wobei die Endabschnitte (3a, 3b) proximal bzw. distal von den Rastelementen (2d) mit der Außenseite (2c) des Kanülenrohres (2) dicht verbunden, vorzugsweise verklebt oder verschweißt, sind.

3. Tracheostomievorrichtung (1) nach Anspruch 2, **dadurch gekennzeichnet, dass** der distale Endabschnitt (3b) des Schlauches (3) in einem Abstand von den am weitesten distal angeordneten Rastelementen (2d) mit dem Kanülenrohr (2) verbunden ist, der mindestens dem Außendurchmesser des Kanülenrohres (2) entspricht und höchstens 30 mm beträgt.

4. Tracheostomievorrichtung (1) nach einem der Ansprüche 1 bis 3, **dadurch gekennzeichnet, dass** der Schlauch (3) aus einem elastischen Kunststoff, vorzugsweise einem Silikon oder einem thermoplastischen Elastomer, hergestellt ist.

5. Tracheostomievorrichtung (1) nach einem der Ansprüche 2 bis 4, **dadurch gekennzeichnet, dass** der zwischen dem proximalen Endabschnitt (3a) und dem distalen Endabschnitt (3b) angeordnete zentrale Abschnitt (3c) des Schlauches (3) gegenüber dem von diesem zentralen Abschnitt (3c) umfassten Abschnitt des Kanülenrohres (2) ein axiales und/oder radiales Übermaß hat, wobei der zentrale Abschnitt (3c) des Schlauches (3) für eine Befüllung mit einem Fluid ausgelegt ist, so dass der zentrale Abschnitt (3c) gegenüber dem darunter befindlichen Kanülenrohr (2) auf ein Übermaß bringbar ist.

6. Tracheostomievorrichtung (1) nach Anspruch 5, **dadurch gekennzeichnet, dass** der Schlauch (3) bei einem Fülldruck von höchstens 100 hPa, vorzugsweise in einem Bereich von 30 hPa bis 50 hPa, einen maximalen Außendurchmesser aufweist, der mindestens das 1,5-fache des Außendurchmesser des darunter befindlichen Kanülenrohrs (2) beträgt.

7. Tracheostomievorrichtung (1) nach einem der Ansprüche 5 oder 6, **dadurch gekennzeichnet, dass** der zentrale Schlauchabschnitt (3c) aus einer inneren Folie und einer äußeren Folie besteht, wobei die innere Folie und die äußere Folie voneinander getrennt sind und die äußere Folie bei einem Innendruck von höchstens 100 hPa, vorzugsweise höchstens 50 hPa, auf einen Durchmesser ausdehnbar ist, der mindestens das 1,5-fache des Außendurchmessers des unter dem zentralen Schlauchabschnitts (3c) angeordneten Kanülenrohrs (2) beträgt, wobei die innere Folie aus einem elastischen Material mit einer geringeren elastischen Dehnung als die äußere Folie hergestellt ist und die innere Folie im Vergleich zu der äußeren Folie ein Übermaß aufweist, wobei vorzugsweise die innere Folie ein High-Volume-Low-Pressure-Cuff ist.

8. Tracheostomievorrichtung (1) nach einem der Ansprüche 1 bis 7, **dadurch gekennzeichnet, dass** die Rastelemente (2d) ein sich in axialer Richtung des Kanülenrohrs (2) wiederholendes Muster aus Vorsprüngen und Vertiefungen bilden, wobei die Rastelemente (2d) mehrere, vorzugsweise zwischen zwei und zehn, in Umfangsrichtung verlaufende und axial äquidistante Rippen und dazwischen liegende Nuten aufweisen.

9. Tracheostomievorrichtung (1) nach einem der Ansprüche 1 bis 8, **dadurch gekennzeichnet, dass** die Rastelemente (2d) sich mindestens über einen Umfangswinkelabschnitt von 30° um das Kanülenrohr (2) herum erstrecken, wobei sich die Rastelemente (2d) vorzugsweise über mindestens zwei einander diametral gegenüberliegende Umfangswinkelabschnitte von jeweils 20° bis 60° um das Kanülenrohr (2) herum erstrecken.

10. Tracheostomievorrichtung (1) nach einem der Ansprüche 1 bis 9, **dadurch gekennzeichnet, dass** ein Kanülenschild (4) mit einer den Schlauch (3) zumindest teilweise umgreifenden Öffnung (4a) vorgesehen ist, deren innerer Rand mit den Rastelementen wahlweise in und außer Eingriff bringbar ist, so dass die axiale Position des Kanülenschildes (4) gegenüber dem Kanülenrohr (2) im Bereich der Rastelemente (2d) vorzugsweise in diskreten Schritten einstellbar und fixierbar ist.

11. Tracheostomievorrichtung (1) nach Anspruch 10, **dadurch gekennzeichnet, dass** das Kanülenschild (4) ein Befestigungsmittel (4b) aufweist, das unter Verformung des Schlauches (3) zusammen mit einem von dem Befestigungsmittel erfassten Schlauchabschnitt in die Rastelemente (2d) eingreift.

12. Tracheostomievorrichtung (1) nach einem der Ansprüche 10 oder 11, **dadurch gekennzeichnet, dass** der Schlauch (3) im Bereich des Kanülenschildes reversibel, vorzugsweise weitestgehend fluiddicht oder gasdicht, in zwei axial getrennte Kammern (3d, 3e) unterteilt ist.

13. Tracheostomievorrichtung (1) nach einem der Ansprüche 1 bis 12, wobei die Tracheostomievorrichtung ein Tracheostomieplatzhalter oder Stomabutton ist, **dadurch gekennzeichnet, dass** an dem distalen Ende (2b) ein Kragen (2e) oder Wulst angeordnet ist, wobei der Kragen (2e) vorzugsweise mit einem Fluid befüllbar und auf ein radiales Übermaß gegenüber dem Außendurchmesser des Kanülenrohrs (2) an dem distalen Ende (2b) bringbar ist.

14. Tracheostomievorrichtung (1) nach Anspruch 13, direkt oder indirekt rückbezogen auf Anspruch 5, **dadurch gekennzeichnet, dass** der Kragen (2e) zusammen mit dem zentralen Abschnitt (3c) des Schlauches (3) befüllbar ist, wobei vorzugsweise der Kragen (2e) eine Materialstärke aufweist, die 3 bis 10-mal höher ist als die Materialstärke des zentralen Abschnittes (3c) des Schlauches (3).

15. Tracheostomievorrichtung (1) nach einem der Ansprüche 13 oder 14, **dadurch gekennzeichnet, dass** distal des Kragens (2e) eine Materialverstärkung, vorzugsweise ein das Kanülenrohr (2) umgebender Flansch (6), zum Abstützen des Kragens (2e) angeordnet ist.

## Claims

1. A tracheostomy device (1), in particular a tracheostomy cannula or tracheostomy placeholder, comprising a cannula tube (2), wherein the cannula tube (2) has a proximal end (2a), a distal end (2b) and an outside (2c), wherein the outside (2c) of the cannula tube (2) in a region arranged centrally between the proximal end (2a) and the distal end (2b) or closer to the proximal end has latching elements (2d) for fixing a cannula shield (4), **characterised in that** a deformable tube (3) which encloses the outside (2c) of the cannula tube (2) covers the latching elements (2d).

2. A tracheostomy device (1) according to claim 1 **characterised in that** the tube (3) has a proximal end portion (3a) and a distal end portion (3b), wherein the end portions (3a, 3b) are sealingly connected, preferably glued or welded, to the outside (2c) of the cannula tube (2) proximally and distally respectively from the latching elements (2d).

3. A tracheostomy device (1) according to claim 2 **characterised in that** the distal end portion (3b) of the tube (3) is connected to the cannula tube (2) at a spacing from the furthest distally arranged latching elements (2d), that corresponds at least to the outside diameter of the cannula tube (2) and is at most 30 mm.

4. A tracheostomy device (1) according to one of claims 1 to 3 **characterised in that** the tube (3) is made from an elastic plastic, preferably a silicone or a thermoplastic elastomer.

5. A tracheostomy device (1) according to one of claims 2 to 4 **characterised in that** the central portion (3c) of the tube (3), that is arranged between the proximal end portion (3a) and the distal end portion (3b), is of an axial or radial oversize with respect to the portion of the cannula tube (2), that is embraced by said central portion (3c), wherein the central portion (3c) of the tube (3) is designed for filling with a fluid so that the central portion (3c) can be brought to an oversize with respect to the cannula tube (2) disposed beneath same.

6. A tracheostomy device (1) according to claim 5 **characterised in that** at a filling pressure of at most 100 hPa, preferably in a range of 30 hPa to 50 hPa, the tube (3) is of a maximum outside diameter which is at least 1.5 time the outside diameter of the cannula tube (2) beneath same.

7. A tracheostomy device (1) according to one of claims 5 or 6 **characterised in that** the central tube portion (3c) comprises an inner film and an outer film, wherein the inner film and the outer film are separate from each other and the outer film at an internal pressure of at most 100 hPa, preferably at most 50 hPa, can be expanded to a diameter which is at least 1.5 times the outside diameter of the cannula tube (2) arranged beneath the central tube portion (3c), wherein the inner film is made from an elastic material with a lower level of elastic stretch than the outer film and the inner film is of an oversize in comparison with the outer film, wherein preferably the inner film is a high-volume low-pressure cuff.

8. A tracheostomy device (1) according to one of claims 1 to 7 **characterised in that** the latching elements (2d) form a pattern comprising projections and recesses, which is repeated in the axial direction of the cannula tube (2), wherein the latching elements (2d) have a plurality of, preferably between two and ten, peripherally extending axially equidistant ribs and grooves disposed therebetween.

9. A tracheostomy device (1) according to one of claims 1 to 8 **characterised in that** the latching elements (2d) extend at least over a peripheral angular portion of 30° around the cannula tube (2), wherein the latching elements (2d) preferably extend over at least two mutually diametrally oppositely disposed peripheral angular portions of 20° to 60° respectively around the cannula tube (2).

10. A tracheostomy device (1) according to one of claims 1 to 9 **characterised in that** there is provided a cannula shield (4) having an opening (4a) which at least partially extends around the tube (3) and the inner edge of which can be selectively brought into and out of engagement with the latching elements so that the axial position of the cannula sheild (4) can be adjusted and fixed preferably in discrete steps with respect to the cannula tube (2) in the region of the latching elements (2d).

11. A tracheostomy device (1) according to claim 10 **characterised in that** the cannula shield (4) has a fixing means (4b) which with deformation of the tube (3) engages into the latching elements (2d) together with a tube portion gripped by the fixing means.

12. A tracheostomy device (1) according to one of claims 10 or 11 **characterised in that** in the region of the cannula shield the tube (3) is subdivided reversibly, preferably very substantially fluid-tightly or gastightly, into two axially separate chambers (3d, 3e).

13. A tracheostomy device (1) according to one of claims 1 to 12 wherein the tracheostomy device is a tracheostomy placeholder or stoma button, **characterised in that** a collar (2e) or bead is arranged at the distal end (2b), wherein the collar (2e) can preferably be filled with a fluid and can be brought to a radial oversize with respect to the outside diameter of the cannula tube (2) at the distal end (2b).

14. A tracheostomy device (1) according to claim 13 directly or indirectly appended to claim 5 **characterised in that** the collar (2e) can be filled together with the central portion (3c) of the tube (3), wherein preferably the collar (2e) is of a material thickness which is 3 to 10 times higher than the material thickness of the central portion (3c) of the tube (3).

15. A tracheostomy device (1) according to one of claims 13 or 14 **characterised in that** arranged distally of the collar (2e) is a material reinforcement, preferably a flange (6) surrounding the cannula tube (2), for supporting the collar (2e).

## Revendications

1. Dispositif de trachéostomie (1), en particulier canule de trachéostomie ou calibreur de trachéostomie, avec un tube de canule (2), dans lequel le tube de canule (2) présente une extrémité proximale (2a), une extrémité distale (2b) et une face externe (2c), dans lequel la face externe (2c) du tube de canule (2) présente, dans une région agencée de manière centrale entre l'extrémité proximale (2a) et l'extrémité distale (2b) ou de manière plus proche de l'extrémité proximale, des éléments d'encliquetage (2d) destinés à l'immobilisation d'une collerette de canule (4), **caractérisé en ce qu'**un flexible (3) déformable entourant la face externe (2c) du tube de canule (2) recouvre les éléments d'encliquetage (2d).

2. Dispositif de trachéostomie (1) selon la revendication 1, **caractérisé en ce que** le flexible (3) présente une section d'extrémité proximale (3a) et une section d'extrémité distale (3b), dans lequel les sections d'extrémité (3a, 3b) sont reliées, de manière préférée collées ou soudées, de manière étanche à la face externe (2c) du tube de canule (2) à proximité ou à distance des éléments d'encliquetage (2d).

3. Dispositif de trachéostomie (1) selon la revendication 2, **caractérisé en ce que** la section d'extrémité distale (3b) du flexible (3) est reliée au tube de canule (2) à une distance des éléments d'encliquetage (2d) agencés de la manière la plus distale qui correspond au moins au diamètre extérieur du tube de canule (2) et représentant au plus 30 mm,.

4. Dispositif de trachéostomie (1) selon l'une quelconque des revendications 1 à 3, **caractérisé en ce que** le flexible (3) est fabriqué à partir d'une matière synthétique élastique, de manière préférée d'un silicone ou d'un élastomère thermoplastique.

5. Dispositif de trachéostomie (1) selon l'une quelconque des revendications 2 à 4, **caractérisé en ce que** la section centrale (3c) du flexible (3) agencée entre la section d'extrémité proximale (3a) et la section d'extrémité distale (3b) présente une cote axiale et/ou radiale en excès par rapport à la section du tube de canule (2) entourée par ladite section centrale (3c), dans lequel la section centrale (3c) du flexible (3) est conçue pour un remplissage avec un fluide, de sorte que la section centrale (3c) peut être amenée à présenter une cote en excès par rapport au tube de canule (2) se trouvant en dessous.

6. Dispositif de trachéostomie (1) selon la revendication 5, **caractérisé en ce que**, pour une pression de remplissage d'au plus 100 hPa, de manière préférée située dans une plage comprise entre 30 hPa et 50 hPa, le flexible (3) présente un diamètre extérieur maximal représentant au moins 1,5 fois le diamètre extérieur du tube de canule (2) se trouvant en dessous.

7. Dispositif de trachéostomie (1) selon la revendication 5 ou 6, **caractérisé en ce que** la section de flexible centrale (3c) est constituée d'un film intérieur et d'un film extérieur, dans lequel le film intérieur et le film extérieur sont séparés l'un de l'autre et le film extérieur peut être dilaté, pour une pression intérieure d'au plus 100 hPa, de manière préférée d'au plus 50 hPa, jusqu'à un diamètre représentant au moins 1,5 fois le diamètre extérieur du tube de canule (2) agencé sous la section de flexible centrale (3c), dans lequel le film intérieur est fabriqué à partir d'une matière élastique présentant une dilatation élastique inférieure à celle du film extérieur et le film intérieur présente une cote en excès par rapport au film extérieur, dans lequel le film intérieur est de manière préférée un manchon basse pression à grand volume.

8. Dispositif de trachéostomie (1) selon l'une quelconque des revendications 1 à 7, **caractérisé en ce que** les éléments d'encliquetage (2d) forment un motif de saillies et de creux se répétant dans la direction axiale du tube de canule (2), dans lequel les éléments d'encliquetage (2d) présentent plusieurs, de manière préférée entre deux et dix, nervures s'étendant dans la direction périphérique et axialement équidistantes et des rainures situées entre lesdites nervures.

9. Dispositif de trachéostomie (1) selon l'une quelconque des revendications 1 à 8, **caractérisé en ce que** les éléments d'encliquetage (2d) s'étendent autour du tube de canule (2) au moins sur une section angulaire périphérique de 30°, dans lequel les éléments d'encliquetage (2d) s'étendent de manière préférée autour du tube de canule (2) sur au moins deux sections angulaires périphériques comprises entre respectivement 20° et 60° et diamétralement opposées l'une à l'autre.

10. Dispositif de trachéostomie (1) selon l'une quelconque des revendications 1 à 9, **caractérisé en ce qu'**une collerette de canule (4) est munie d'un orifice (4a) entourant au moins partiellement le flexible (3) et dont le bord intérieur peut être mis en prise et hors de prise de manière sélective avec les éléments d'encliquetage, de sorte que la position axiale de la collerette de canule (4) par rapport au tube de canule (2) dans la région des éléments d'encliquetage (2d) peut être ajustée et immobilisée de manière préférée par incréments discrets.

11. Dispositif de trachéostomie (1) selon la revendication 10, **caractérisé en ce que** la collerette de canule (4) présente un moyen de fixation (4b) qui, lors de la déformation du flexible (3), vient en prise dans les éléments d'encliquetage (2d) conjointement avec une section de flexible saisie par le moyen de fixation.

12. Dispositif de trachéostomie (1) selon la revendication 10 ou 11, **caractérisé en ce que**, dans la région de la collerette de canule, le flexible (3) est divisé de manière réversible, de manière préférée de manière essentiellement étanche aux fluides ou aux gaz, en deux chambres (3d, 3e) séparées de manière axiale.

13. Dispositif de trachéostomie (1) selon l'une quelconque des revendications 1 à 12, dans lequel le dispositif de trachéostomie est un calibreur de trachéostomie ou un bouton de stomie, **caractérisé en ce qu'**un col (2e) ou un bourrelet est agencé au niveau de l'extrémité distale (2b), dans lequel le col (2e) peut de manière préférée être rempli avec un fluide et peut être amené au niveau de l'extrémité distale (2b) avec une cote radiale en excès par rapport au diamètre extérieur du tube de canule (2).

14. Dispositif de trachéostomie (1) selon la revendication 13, dépendant directement ou indirectement de la revendication 5, **caractérisé en ce que** le col (2e) peut être rempli conjointement avec la section centrale (3c) du flexible (3), dans lequel le col (2e) présente de manière préférée une épaisseur de matière qui est entre 3 et 10 fois supérieure à l'épaisseur de matière de la section centrale (3c) du flexible (3).

15. Dispositif de trachéostomie (1) selon la revendication 13 ou 14, **caractérisé en ce qu'**un renfort de matière, de manière préférée une bride (6) entourant le tube de canule (2), est agencé de manière distale par rapport au col (2e) afin de soutenir le col (2e).
